# EUROPÄISCHE PATENTANMELDUNG

(11) **EP 3 632 895 A1**
(43) Veröffentlichungstag der Anmeldung: **08.04.2020**
(21) Anmeldenummer: 18197951.9
(22) Anmeldetag: 01.10.2018
(51) Int. Cl.: C07C 319/20, C07C 231/06, C07C 237/06, C07C 323/58

(54) **SALZFREIE HERSTELLUNG VON AMINOSÄUREN AUS IHREN AMINONITRILEN**

(71) Anmelder: Evonik Operations GmbH, 45128 Essen (DE)
(72) Erfinder: ROST, Daniel, 68163 Mannheim (DE); FISCHER, Daniel, Midlothian, VA 23113 (US); BILZ, Jürgen, 63579 Freigericht (DE); RONNEBURG, Axel, 63456 Hanau (DE); JÄGER, Barbara, 63533 Mainhausen (DE); JAKOB, Harald, 63594 Hasselroth (DE); REUS, Christian, 63579 Freigericht (DE); HASSEBERG, Hans-Albrecht, 63584 Gründau-Lieblos (DE)
(74) Vertreter: Evonik Patent Association

(57) **Zusammenfassung**

Die Erfindung betrifft ein Verfahren zur Herstellung von α-Aminosäureamid, insbesondere von Methioninamid, durch Hydrolyse des entsprechenden α-Aminosäurenitrils, insbesondere von Methioninnitril, in Gegenwart eines Ketons und eines sekundären Amins als Katalysator, sowie ein Verfahren zur Herstellung der entsprechenden α-Aminosäure, insbesondere von Methionin, unter Einbeziehung dieses Verfahrens.

## Beschreibung

Aminosäuren haben insbesondere aufgrund ihrer Funktion als Eiweißbausteine für die Ernährung von Tier und Mensch eine fundamentale Bedeutung Futtermittel in der Nutztierhaltung werden daher zusätzlich mit Aminosäuren, z. B. DL-Methionin und L-Lysin, angereichert, wodurch deren Nährwert erhöht wird.
DL-Methionin ist eine essenzielle Aminosäure, die mit der Nahrung aufgenommen werden muss. Als Futtermittelzusatz trägt sie zu einer effizienten, gesunden und umweltschonenden Ernährung von landwirtschaftlichen Nutztieren, insbesondere von Geflügel und Schweinen, bei. Sie ist damit auch ein wichtiger Baustein, wenn es um die nachhaltige Versorgung einer wachsenden Weltbevölkerung mit tierischem Protein geht. Somit kommt einer kostengünstigen und auch großindustriell gut durch führbaren Synthesemethode für DL-Methionin eine hohe Bedeutung zu.

### Stand der Technik:

Im industriellen Maßstab wird Methionin chemisch über die Bucherer-Bergs-Reaktion hergestellt, die eine Variante der Strecker-Synthese darstellt. Dabei werden die Ausgangssubstanzen 3-Methylmercaptopropanal (hergestellt aus 2-Propenal und Methylmercaptan), Blausäure (Cyanwasserstoff), Ammoniak und Kohlendioxid zum 5-(2-Methylmercaptoethyl)-hydantoin (Methioninhydantoin) umgesetzt. Die Hydrolyse des Hydantoins erfordert harsche Bedingungen und stöchiometrische Mengen an einer Base, meist Natriumhydroxid oder Kaliumhydroxid bzw. Kaliumcarbonat. Nach einem altbekannten Verfahren wird Methionin im Hydrolysat durch Neutralisation mit Schwefelsäure aus seinem Natriumsalz freigesetzt, welches als Präzipitat aus der Natriumsulfat enthaltenden Mutterlauge abfiltriert werden kann. Das Koppelprodukt Natriumsulfat muss anderweitig verwertet oder entsorgt werden.
Nach dem bekannten Degussa-Kaliumcarbonatkreislaufverfahren wird Methionin schließlich durch Behandlung des Hydrolysats mit Kohlendioxid aus seinem Kaliumsalz freigesetzt, wobei das Methionin-Präzipitat aus der Kaliumcarbonat und Kaliumhydrogencarbonat enthaltenden Mutterlauge abfiltriert werden kann (US 5,770,769). Letzteres kann zwar zurückgewonnen werden, doch ist dafür ein Kreislauf einer großen Menge salzhaltiger Lösung erforderlich.
Bedingt durch den mit der Zeit ansteigenden Nebenproduktpegel in der zu recyclierenden wässrigen Mutterlauge ist es notwendig nicht unerhebliche Mengen der Mutterlauge aus dem Verfahren auszuschleusen, was den zusätzlichen Aufwand einer Aufarbeitung oder Entsorgung mit sich bringt.
Zum anderen sind die Bedingungen der Hydantoinbildung und der Hydantoinhydrolyse mit Temperaturen von bis zu über 200 °C harsch und energieintensiv, so dass weiterhin Bedarf an einer großindustriell durchführbaren Methode bestand, die die genannten Nachteile nicht oder kaum mehr aufweist.

Eine bekannte Alternative zur Bucherer-Bergs-Reaktion stellt der Herstellungsweg via Methioninnitril, dem Aminonitril von 3-Methylmercaptopropionaldehyd (MMP-AN) und dessen anschließende Verseifung zu Methionin mit Hilfe stöchiometrischer Mengen Kalilauge oder Schwefelsäure nach Strecker dar. Aber auch diese Route macht der hohe stöchiometrische Salzabfall, der durch die ebenfalls nötige Neutralisation bedingt ist, unattraktiv für ein großindustrielles Herstellungsverfahren.
Alternativ können die Aminonitrile in Gegenwart eines Ketons wie z.B. Aceton und einer starken Hydroxidbase wie z.B. NaOH als Katalysator mit hoher Selektivität schon bei Raumtemperatur in die entsprechenden alpha-Aminoamide umgewandelt werden (z.B. Bull. Soc. Chim. Fr. 1978, 3-4, II-177 bzw. DE2637204). Eine anschließende Verseifung des Methioninamids mittels Alkalien liefert unter vergleichsweise milden Bedingungen von z:B. 100 - 120°C das Alkalimetallsalz des Methionins (z.B. gemäß WO 94/08957 A1, Beispiel 18), aus dem wiederum durch Neutralisation mit Säure Methionin erst freigesetzt werden muss unter Bildung entsprechender Salze als unerwünschtem Koppelprodukt (Schema 1). Die Ausbeuten an Methioninamid bzw. Methionin liegen bei ca. 95- 97 % und sind damit zwar hoch jedoch nicht quantitativ.
Aber auch wenn die anschließende Verseifung des Amids direkt zur Carbonsäure über ein Neutralverseifungsverfahren mit einem heterogenen Katalysator (z.B. TiO₂) angewendet wird (Schema 1, z.B. DE 60127538 T2), so ist diese Route dennoch nicht salzfrei, da 10-20 mol% einer starken Base wie z.B. NaOH in der Amidbildungsreaktion eingesetzt werden müssen. Diese Base muss durch physikalisch-chemische Methoden wie z.B. lonenaustauscherharze wieder abgetrennt werden.

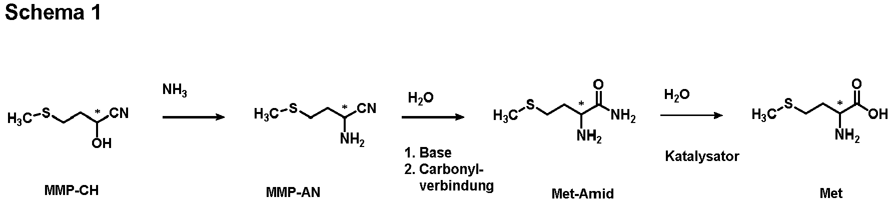

Alternativ ist die Verwendung von polymer gebundenen Hydroxidbasen bekannt (z.B. FR2785609). Diese haben jedoch den Nachteil, dass sie häufig regeneriert werden müssen, was wiederum zu Salzabfall führt.
Darüberhinaus sind im Stand der Technik mehrere Verfahrensvarianten für andere Aminosäureamide außer Methioninamid offenbart, die sich auch die carbonylkatalysierte alkalische Hydrolyse der betreffenden Aminonitrile zu Nutze machen.

JP2001-199947A offenbart ein Verfahren zur Hydrolyse von Aminosäurenitril zu Aminosäureamiden bzw. Aminosäuren. Dabei wird zunächst aus einem Cyanhydrin mit Ammoniak ein entsprechendes Aminonitril hergestellt. Dabei werden Alkalimetallhydroxide wie Natriumhydroxid oder Kaliumhydroxid in einem Vorzugsbereich von 0,01 bis 0,10 Molequivalente (Moleq) pro ein Moleq Cyanhydrin und Methylethylketon oder Aceton als Carbonylkatalysator verwendet und in einem Temperaturbereich von 0 bis 20 °C zur Reaktion gebracht. Alaninamid, Valinamid und Phenylglycinamid wurden auf diese Weise erhalten. Die Reaktion von Methioninnitril wurde nicht explizit untersucht.

JP 2001- 247529 A offenbart ebenfalls ein Verfahren zur Hydrolyse von Aminosäurenitril zu Aminosäureamiden. Im Beispiel beschrieben ist nur ein Verfahren zur Herstelllung von tert.Leucinamid. Dabei werden Alkalimetallhydroxide wie Natriumhydroxid oder Kaliumhydroxid vorzugsweise in einem überaus breiten Vorzugsbereich von 0,01-10 Moleq (0,5 Moleq im Beispiel) pro ein Moleq Aminonitril und niedermolekulare Ketone, insbesondere Aceton als Carbonylkatalysator verwendet, bei Temperaturen von ca. 20 - 30°C und teils sehr langen Reaktionszeiten von 1-100 Stunden. Im Falle von tert-Leucinamid wurden auf diese Weise jedoch nur 83 bis 91% Ausbeute erhalten. Ein Verfahren zur Herstellung von Methioninamid ist nicht offenbart. Der relativ hohe Basenanteil von z.B. 0,5 Moleq NaOH erfordert wiederum einen relativ hohen Säureanteil, der zur Neutralisation und Isolierung von neutralem Methionin gebraucht wird. Das führt zu erheblichem Anfall von Salz als Beiprodukt, was unter ökonomischen wie ökologischen Gesichtspunkten nachteilig ist.

JP5613162B A offenbart schließlich ein Verfahren zur Herstellung des Salzes von 2-Aminobuttersäureamid mit einer anorganischen Säure durch Hydrolyse von 2-Aminobuttersäurenitril mittels einer starken anorganischen Base und einem Keton (Aceton oder Methylethylketon) als Lösungsmittel und damit in großer Menge. Dabei werden Alkalimetallhydroxide wie Natriumhydroxid oder Kaliumhydroxid in einem Vorzugsbereich von 0,005-0,10 Mol pro ein Mol Cyanhydrin und Methylethylketon oder Aceton als Carbonylkatalysator verwendet und bei einer Temperatur von 20 °C 7 Stunden lang (Beispiele) zur Reaktion gebracht. Am Ende wird durch Zugabe einer starken Mineralsäurelösung das Mineralsäuresalz des 2-Aminobuttersäureamid erzeugt. Auch diese Variante ist aufgrund des hohen Anfalls von Salz als Beiprodukt nachteilig.

CN102070473 A offenbart schließlich ein Verfahren zur Herstellung von 2-Amino-3-methylbuttersäureamid (Valinamid) durch Hydrolyse von 2- Amino-3-methyl-buttersäurenitril (Valinnitril) mittels einer starken anorganischen Base und einem Keton (Aceton oder Methylethylketon) als Carbonylkatalysator. Dabei werden Natriumhydroxid, Kaliumhydroxid oder Bariumhydroxid im Gewichtsverhältnis von 0,01-1,0 :1 und bevorzugt 0,05-0,50 :1 bezogen auf die Menge an Keton und das Keton im Verhältnis von 0,5-2,0 Moleq pro 1 Moleq Valinnitril und bei einer Temperatur von -10 bis 10 °C 5-8 Stunden lang (Beispiele) zur Reaktion gebracht. Am Ende wird durch Extraktion mit organischen Lösungsmittel (Chloroform) das Valinamid aus der wässrigen Salzlösung entfernt, was mit verringerten Ausbeuten von nur ca. 63-85 % einhergeht. Auch diese Variante ist aufgrund des hohen Anfalls von Salz als Beiprodukt und der Verwendung von organischem Lösungsmittel nachteilig.

### Aufgabe:

Die der vorliegenden Erfindung grundsätzlich zugrundeliegende Aufgabe war demzufolge die Bereitstellung eines möglichst einfachen chemischen Verfahrens zur Herstellung von D,L-Aminosäuren, insbesondere von D,L- Methionin, bei dem weniger harsche Bedingungen als in der klassischen Methode über das Hydantoin ermöglicht werden. Dabei sollte gleichzeitig der Zwangsanfall von Salzen als Koppelprodukt der klassischen Verfahren vermieden werden. Eine Teilaufgabe in diesem Zusammenhang war es, ein Verfahren zur salzfreien Herstellung von D,L-Aminosäureamiden, insbesondere von D,L- Methioninamid bereitzustellen, welches dann durch Kopplung mit einem Verfahren zur Neutralverseifung von D,L-Aminosäureamiden zu salzfreien Gesamtverfahren zur Herstellung von D,L-Aminosäuren, insbesondere von D,L- Methionin ergänzbar ist.

### Lösung:

Diese grundlegende Teilaufgabe wurde gelöst durch das Bereitstellen eines Verfahrens zur Herstellung von α-Aminosäureamid, insbesondere von Methioninamid, durch Hydrolyse von α-Aminosäurenitril, insbesondere von Methioninnitril, in Gegenwart eines Ketons und eines sekundären Amins als Katalysator.

Unerwarteterweise wurde gefunden, dass nicht nur die in der Literatur beschriebenen HydroxidBasen die Umsetzung von Aminonitrilen in Gegenwart von Ketonen, wie Aceton, zu den entsprechenden Aminoamiden katalysieren, sondern auch Amine, speziell sekundäre Amine mit geringem Molekulargewicht, wie z.B. Dimethylamin, Diethylamin, Pyrrolidin. Diese haben den Vorteil, dass sie durch eine Destillation zusammen mit Aceton abgetrennt werden können, wodurch in Kombination mit einer Hydrolyse der so erhaltenen Amide mit Hilfe eines neutralen z.B.TiO₂-haltigen Katalysators eine komplett salzfreie Route zu Aminosäuren insbesondere Methionin aus den entsprechenden Aldehyden ermöglicht wird.
Die eigentlich durch den Austausch von Ammoniak gegen das sekundäre Amin (während der Hydrolysereaktion) zu erwartenden N,N-Dialkylmethioninamide treten außerdem dabei nicht auf, Dies ist überaus überraschend, weil in der einschlägigen Fachliteratur beschrieben ist, dass Dialkylamine wie Dimethylamin mit Aldehyden bzw. Ketonen und Blausäure die entsprechenden tertiären N,N-Dialkylaminonitrile bilden, z.B. gemäß A.Commeyras et al., Informations Chimie (1976), Nr.158; 199-207 am Beispiel von Acetaldehyd, Dimethylamin und Blausäure, so dass bei Einsatz von Dialkylaminen in der carbonylkatalysierten Hydrolyse von α-Aminonitril genau deren Bildung über einen Austausch des Ammoniaks im Methioninnitril gegen Dimethylamin (Bildungs/Zerfallsgleichgewicht), zu erwarten gewesen wäre. Da die in der Folge entstehenden N,N-Dimethylaminosäuren jedoch unerwünscht und im Falle ihrer Entstehung schwer abtrennbar sind, hätte der Fachmann die Verwendung von Dialkylaminen als Basen im Zusammenhang mit der Hydrolyse von Methioninnitril von Anfang an gar nicht in Betracht gezogen. So wurden, wie die Umsetzungen bei 35 °C für 2,5 h mit Hilfe der sekundären Amine Dimethylamin bzw. Pyrrolidin als Basenkatalysator und Aceton als Carbonylkatalysator in den Beispielen 1 und 2 zeigen, Ausbeuten von 88 bzw. 92 % Methioninamid erhalten.

Überraschend ist es auch, dass im Gegensatz dazu primäre Amine, obwohl ähnliche Basizität habend wie sekundäre Amine, deutlich schlechter katalysieren als die erfindungsgemäß verwendeten sekundären Amine, wie die Umsetzungen mit Hilfe der primären Amine Spermidin und 1,2-Diaminocyclohexan zeigen (Vergleichsbeispiel 7 und 8) mit Umsätzen von nur 38 bis 53 % bei 35 °C für 2,5 h.

Die genannte Aufgabe wurde insbesondere gelöst durch Bereitstellen eines Verfahrens zur Herstellung von Methioninamid gekennzeichnet durch folgende Schritte:
a. Herstellung von Methioninnitril durch Umsetzung von Methylmercaptopropionaldehyd (MMP) mit Blausäure und Ammoniak und/oder durch Umsetzung des entsprechenden MMP-Cyanhydrins (MMP-CH) mit Ammoniak zu einem Methioninnitril enthaltendem Reaktionsgemisch,
b. optional wenigstens teilweise Abtrennung von restlichem Ammoniak aus dem Reaktionsgemisch aus a.,
c. Hydrolyse des Methioninnitrils zum Methioninamid im Reaktionsgemisch aus a. oder b. in Gegenwart eines Ketons und eines sekundären Amins als Katalysator und
d. optional Abtrennung des Ketons und des sekundären Amins aus dem Reaktionsgemisch aus c.

Dabei werden die in Schritt a. verwendeten Reaktanden MMP und HCN vorzugsweise äquimolar, jedoch mit bis zu 1,03 Moleq HCN bezogen auf 1 Moleq MMP eingesetzt. Ammoniak wird in Schritt a. dabei bevorzugt in Mengen von 1 bis 10 Moleq Ammoniak pro 1 Moleq der molaren Summe aus Methylmercaptopropionaldehyd und MMP-Cyanhydrin eingesetzt. Wird also ausschließlich von vorgebildetem MMP-CH ausgegangen bezieht sich die Ammoniakmenge ausschließlich auf MMP-CH. Wird im anderen Extremfall ausschließlich von MMP und HCN ausgegangen, so bezieht sich die Ammoniakmenge ausschließlich auf MMP.
Ein solches Verfahren bietet den großen Vorteil, dass man ausgehend von den üblichen Rohstoffen der Methioninsynthese, MMP, HCN und Ammoniak unter Verwendung kostengünstiger sekundärer Amine, die obendrein recyclierbar sind, zu Methioninamid gelangt, der direkten Vorstufe des Futtermitteleinsatzstoffes Methionin.

Die oben genannte grundsätzliche Aufgabe wurde des Weiteren gelöst durch Bereitstellen eines Verfahrens zur Herstellung des eigentlichen kommerziell verwertbaren Endproduktes Methionin gekennzeichnet durch folgende Schritte:
a. Herstellung von Methioninnitril durch Umsetzung von Methylmercaptopropionaldehyd (MMP) mit Blausäure und Ammoniak und/oder durch Umsetzung des entsprechenden MMP-Cyanhydrins (MMP-CH) mit Ammoniak zu einem Methioninnitril enthaltenden Reaktionsgemisch,
b. optional wenigstens teilweise Abtrennung von restlichem Ammoniak aus dem Reaktionsgemisch aus a.,
c. Hydrolyse von Methioninnitril zum Methioninamid im Reaktionsgemisch aus a. oder b. in Gegenwart eines Ketons und eines sekundären Amins als Katalysator,
d. optional wenigstens teilweise Abtrennung des Ketons und des sekundären Amins aus dem Reaktionsgemisch aus c. und
e. anschließender Hydrolyse des Methioninamids zum Methionin im Reaktionsgemisch aus c. oder d. in Gegenwart eines sauren, basischen oder neutralen Katalysators.

In diesem Zusammenhang bevorzugt werden Verfahren der vorstehend bezeichneten Art, dadurch gekennzeichnet, dass bei der Umsetzung gemäß Schritt a. 1 bis 10 Moleq Ammoniak eingesetzt werden bezogen auf die molare Summe aus Methylmercaptopropionaldehyd und MMP-Cyanhydrin, so wie weiter oben angegeben, jedoch vorzugsweise 2 bis 8 Moleq Ammoniak und besonders bevorzugt 4 bis 7 Moleq Ammoniak. Insbesondere die Ammoniaküberschüsse gewährleisten, dass nur sehr wenig, nämlich ca. 1 Mol% des dimeren Iminodinitrilproduktes als Nebenprodukt entstehen. Die günstigsten Reaktionstemperaturen liegen bei 50 bis 100 °C.

Des Weiteren bevorzugt werden Verfahren der vorstehend bezeichneten Art, dadurch gekennzeichnet, dass bei der Umsetzung gemäß Schritt a. wäßriger Ammoniak in einer Konzentration von 25 bis 80 Gew.-%, vorzugsweise von 30 bis 60 Gew.-% eingesetzt wird. In der Regel genügt es mit ca. 32 Gew.-% Ammoniak zu arbeiten, was den zusätzlichen Vorteil bietet, dass der eingesetzte Ammoniak dabei noch bei Normaldruck zu handhaben ist. Auch die höhere Ammoniakkonzentration trägt zu einer Verringerung der Iminodinitrilbildung bei.

Auch bevorzugt werden Verfahren der vorstehend bezeichneten Art, dadurch gekennzeichnet, dass die optionale Abtrennung von restlichem Ammoniak gemäß Schritt b. bei einem Druck von 0,01 bis 3 bara und einer Temperatur von 20 bis 70 °C, vorzugsweise von 0,1 bis 1 bara und einer Temperatur von 25 bis 60 °C erfolgt. Hierdurch werden die Volumenströme im hinteren Verfahrensabschnitt entlastet und der Reinigungsaufwand für den so isolierten recyclierbaren Ammoniak verringert. Dabei wird die noch heiße wässrig-ammoniakalische Lösung durch zumindest teilweise Entspannung von der Reaktionstemperatur von ca. 50 bis 100°C auf Temperaturen bis max. 40 bis 70 °C abgekühlt.

Ebenfalls bevorzugt werden Verfahren der vorstehend bezeichneten Art, dadurch gekennzeichnet, dass bei der Hydrolyse des Methioninnitrils, im Speziellen gemäß Schritt c., als sekundäres Amin Dimethylamin, Diethylamin, Dipropylamin, Diisopropylamin, Pyrrolidin, 4-Piperidon oder Triacetonamin eingesetzt werden, ganz besonders bevorzugt jedoch Dimethylamin. Die genannten Amine sind allesamt kommerziell erhältlich und sind aus dem Reaktionsgemisch vergleichsweise gut abtrennbar, in der Regel durch Destillation.

Wie der Vergleich der Hydrolyseergebnisse der erfindungsgemäßen Umsetzung von Methioninnitril mit den sekundären Aminen (DMA, Pyrrolidin, 4-Piperidon) und Aceton als Carbonylkatalysator (Beispiele: 1, 2, 6) mit den herkömmlichen Umsetzungen von Methioninnitril mit den Alkalibasen (in Form von NaOH) und Aceton/4-Piperidon/Cyclohexanon als Carbonylkatalysator zeigt (Beispiele: 3, 9, 10, erzielt das erfindungsgemäße Verfahren etwa gleiche bis sogar deutlich höhere Ausbeuten von bis zu ca. 88 bis 92 % im Vergleich zur herkömmlichen Verfahrensweise mit nur 78,6 bis 84,6 %. Dazu kommt noch der große Vorteil, dass die sekundären Amine leicht abtrennbar sind und die verfrühte Weiterreaktion des Amids zur Aminosäure praktisch nicht erfolgt, also im Falle von Methionin keine Methioninatanteile, wie Natriummethioninat, in der Amidstufe auftreten, welche dem Ziel eines salzfreien Verfahrens entgegenstehen würden.

Weiterhin bevorzugt werden Verfahren der vorstehend bezeichneten Art, dadurch gekennzeichnet, dass bei der Hydrolyse des Methioninnitrils, im Speziellen gemäß Schritt c., als Keton Aceton, Methyl-ethylketon, Diethylketon, Methyl-propylketon, Methyl-isopropylketon, Ethyl-propylketon, Cyclopentanon, Cyclohexanon oder 4-Piperidon, vorzugsweise Aceton, Methyl-ethylketon oder 4-Piperidon eingesetzt werden. Alle diese Carbonylverbindungen sind kommerziell problemlos erhältlich. Der Preis ist dabei sekundär, weil diese Carbonylverbindungen als Katalysatoren eingesetzt werden, d.h. bis auf kleinere Verluste nicht verbraucht sondern zurückgewonnen und wiederverwendet werden.
Die Ketone bilden mit vorhandenem Methioninamid in Anteilen von ca.1-3 Mol % Imidazolidinone, im Falle von Aceton das 2,2-Dimethyl- 5-(2'-methylthio) ethylimidazolidin-4-on (IM2):

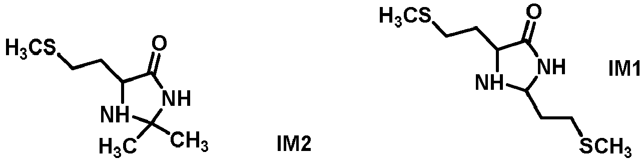

Das analog in noch geringeren Anteilen aus kleinen vorhandenen Anteilen von MMP mit Methioninamid gebildete Imidazolidinon IM1 war hier nicht nachweisbar. Diese Nebenprodukte werden aber in der anschließenden Hydrolyse des Methioninamids ebenfalls problemlos zu Methionin hydrolysiert, so dass dadurch keine Ausbeuteverluste entstehen.

Auch bevorzugt werden Verfahren der vorstehend bezeichneten Art, dadurch gekennzeichnet, dass im Falle der Verwendung von 4-Piperidon als Keton kein weiteres Keton oder im Falle der Verwendung von 4-Piperidon als sekundäres Amin kein weiteres sekundäres Amin verwendet wird. Die erfindungsgemäße Verwendbarkeit der doppelten Funktionalität - Keton und sekundäres Aminin einem Molekül trägt zur deutlichen Vereinfachung des erfindungsgemäßen Verfahrens bei (vgl. Beispiel 6).

Dabei ebenfalls besonders bevorzugt werden Verfahren der vorstehend bezeichneten Art, dadurch gekennzeichnet, dass die Hydrolyse des Methioninnitrils in Schritt c. bezogen auf das Nitril in Gegenwart von 15 bis 50 Moleq H₂O, 0,1 bis 1,5 Moleq Keton, 0 bis 9 Moleq NH₃, 0,1 bis 2,0 Moleq sekundäres Amin sowie bei einer Temperatur zwischen 10 und 60°C und bei einer Reaktionszeit zwischen 10 und 180 Minuten durchgeführt wird.

Dabei vorteilhaft wird die Abtrennung des Ketons und des sekundären Amins gemäß Schritt d. bei einem Druck von 0,001 bis 1 bara und einer Temperatur von 20 bis100 °C, vorzugsweise von 0,01 bis 0,9 bara und einer Temperatur von 25 bis 80 °C durchgeführt, weil so die maximale Ausbeute bei geringsten Nebenproduktanteilen und vergleichsweise kurzen Reaktionszeiten erhalten wird. Dies ist problemlos möglich, z.B. über eine einfache Destillation, wie Beispiel 5 deutlich macht. Besonders vorteilhaft ist auch eine Verfahrensweise, dadurch gekennzeichnet, dass das gemäß Schritt d. abgetrennte Keton und sekundäre Amin zur Hydrolyse des Methioninnitrils in Schritt c. zurückgeführt werden, und so Verluste und Kosten minimiert werden. Dies kann unmittelbar oder wenn zweckmäßig nach einer gewissen Zwischenreinigung z.B. durch Destillation geschehen.

Im letzten Schritt bei der Hydrolyse des Methioninamids zum Methionin können sowohl saure als auch alkalische oder neutrale Katalysatoren verwendet werden.
Bei der Hydrolyse des Methioninamids zum Methionin gemäß Schritt e. kann als saurer Katalysator eine starke Mineralsäure, vorzugsweise Salzsäure, Schwefelsäure oder Phosphorsäure verwendet werden, welche allesamt sehr wirkungsvoll und relativ kostengünstig erhältlich sind. Allerdings führt die mineralsaure Hydrolyse primär zu Methionin-Mineralsäuresalzen, wodurch in diesem Fall zur Isolierung von freiem Methionin wiederum eine Neutralisation mit Base unter Salzanfall nötig wäre. Bei Verwendung eines basischen Katalysator bei der Hydrolyse des Methioninamids gemäß Schritt e. wird vorzugsweise eine Alkali- oder Erdalkali- Base verwendet, insbesondere NaOH, KOH, Mg(OH)₂, Ca(OH)₂ oder Ba(OH)₂. Vorteilhaft dabei ist die schnelle und vollständige Hydrolyse bei moderaten Temperaturen. Nachteilig ist jedoch dabei, dass das Methionin zunächst als Alkali- oder Erdalkalisalz anfällt, aus dem erst wieder durch Neutralisation mit Säure das Methionin freigesetzt werden muss, was zwangsläufig wiederrum zu einem Salzanfall führt. Vorteilhaft ist die basische Hydrolyse dagegen, wenn sowieso ein Alkali- oder Erdalkalimethioninat als Produktform gewünscht ist, da diese dann auf direktem Weg als Endprodukt erhältlich ist.
Die besonders bevorzugte Verfahrensweise ist daher die Hydrolyse des Methioninamids zum Methionin mit Hilfe von neutralen Katalysatoren, weil hier der Salzanfall komplett vermieden werden kann.

Als neutraler Katalysator bei der Hydrolyse des Methioninamids gemäß Schritt e. können wiederum Titandioxide verwendet werden. Durch derartige Verfahren werden in vorteilhafter Weise die Bildung von Salzabfallstoffen vermieden und es wird ein insgesamt salzfreies Verfahren zur Herstellung von Methionin bereitgestellt, was von erheblichem Vorteil ist, wie eingangs dargestellt. Das dabei primär entstehende Ammoniummethioninat kann anschließend leicht thermisch gespalten werden in Methionin, das dabei als Kristallisat anfällt und flüchtigen Ammoniak, der leicht abgetrennt und z.B. in die Aminonitrilsynthesestufe zurückgeführt werden kann.

Ebenso wurde in diesem Zusammenhang gefunden, dass unter den vielen Titanoxiden, die für den Hydrolyseschritt vom Methioninamid zum Methionin Ammoniumsalz getestet wurden, der kommerziell erhältliche Hombikat F01 (Sachtleben/Huntsman, pH-Wert = 6, spezifische Oberfläche = 350 m²/g, Titandioxidgehalt (geglüht) = 88 %, enthält 0,5% Schwefelsäure)) die besten Resultate mit quantitativem Umsatz/Ausbeute bei kurzer Reaktionsdauer ergibt.
Dabei vorteilhaft wird die Hydrolyse des Methioninamids gemäß Schritt e. bei Temperaturen von 80-180°C, vorzugsweise von 100-140°C durchgeführt. Dadurch wird insbesondere eine vollständige Umsetzung erreicht und damit ein qualitativ hochwertiges von Anfang an sehr reines Endprodukt.

Somit ist es gelungen ein technisches Herstellungsverfahren für Methionin bereitzustellen, das ausgehend von den verfügbaren Ausgangsstoffen MMP, Blausäure und Ammoniak über die milde carbonylkatalysierte Hydrolyse zum Methioninamid bei deutlich niedrigeren Temperaturen als beim derzeitigen Standardverfahren über die Hydantoin-Hydrolyse in hohen Ausbeuten und bei einfacherer Verfahrensführung zum Methionin führt.

Da das Prinzip des erfindungsgemäßen Verfahrens zur Herstellung von Methioninamid bzw. Methionin auch auf andere α-Aminosäuren anwendbar ist, ist auch der Gegenstand der Erfindung generell ein Verfahren zur Herstellung eines α-Aminosäureamids sowie der entsprechenden α-Aminosäure.

Gegenstand der Erfindung ist damit auch ein Verfahren zur Herstellung eines α-Aminosäureamids durch Hydrolyse des entsprechenden α-Aminosäurenitrils in Gegenwart eines Ketons und eines sekundären Amins als Katalysator, welches die eingangs am Beispiel des Verfahrens zum Methioninamid/Methionin beschriebenen Vorteile aufweist. Vorzugsweise handelt es sich bei der α-Aminosäure dabei um Alanin, Homoserin, Serin, Threonin, Valin, Leucin, Isoleucin, Lysin, Methionin, Tryptophan, Histidin, Phenylalanin, Glycin oder Tyrosin.
Ganz analog werden auch hier erfindungsgemäß bei der Hydrolyse des α-Aminosäurenitrils als sekundäres Amin, Dimethylamin, Diethylamin, Dipropylamin, Diisopropylamin, Pyrrolidin oder 4-Piperidon eingesetzt und entsprechend als Keton Aceton, Methyl-ethylketon, Diethylketon, Methyl-propylketon, Methyl-isopropylketon oder Ethyl-propylketon.

Das auf diese Weise erfindungsgemäß bereitgestellte Verfahren zur Herstellung einer α-Aminosäure umfasst in einem ersten Schritt die Hydrolyse des entsprechenden α-Aminosäurenitrils zum jeweiligen α-Aminosäureamid in Gegenwart eines Ketons und eines sekundären Amins als Katalysator und in einem zweiten Schritt die Weiterhydrolyse des α-Aminosäureamids zur entsprechenden α-Aminosäure mit einem der weiter oben anhand von Methionin dargestellten Verfahren.

### Beispiele:

### Analytische Methoden

Die chromatographischen Untersuchungen (MMP-Cyanhydrin, MMP, Methionin, Methioninamid, Methioninnitril, IM1, IM2) wurden mittels HPLC der Firma JASCO an einer RP-18 Säule (250 x 4,6 mm; 5 µm) mit anschließender UV Detektion bei 210 nm durchgeführt. Als Laufmittel diente ein phosphorsaures Acetonitril-Wasser-Gemisch (3,3 g H₃PO₄, 6,8 g Acetonitril, 89,9 g H₂O). Bei einem Fluss von 1 mL/min wurden 10 µL der jeweiligen Probelösung (50 mg Probe in 25 mL H₂O) injiziert. Die Kalibrierung erfolgte im Vorfeld durch die Injektion geeigneter Kalibrierlösungen, die Auswertung erfolgte durch Peakflächenvergleich mittels der externen Standardmethode. Die Durchführung der Standardmethode ist dem Fachmann bekannt.

### Beispiel 1: Hydrolyse von MMP-Aminonitril mit Dimethylamin (DMA) und Aceton

In einem 100 ml Schott-Glasdruckgefäß mit Magnetrührung wurden 34 g VE-Wasser, 5,2 g MMP-AN (w/w 80%; 32 mmol, 1 Moleq), 5,0 g Dimethylamin (w/w 40%; 44 mmol,1,4 Moleq) und 2,3 g (39 mmol, 1,2 Moleq) Aceton vorgelegt. Die Emulsion wurde bei 500 U/min und +35°C im Wasserbad 2,5 h gerührt. Es konnte eine Ausbeute von 4,1 g (28 mmol, 88%) Methioninamid via HPLC detektiert werden.

### Beispiel 2: Hydrolyse von MMP-Aminonitril mit Pyrrolidin und Aceton

In einem 100 ml Schott-Glasdruckgefäß mit Magnetrührung wurden 17,5 g VE-Wasser, 5,2 g MMP-AN (w/w 80%; 32 mmol, 1,0 Moleq), 2,9 g Pyrrolidin (41 mmol, 1,3 Moleq) und 2,4 g (41 mmol, 1,3 Moleq) Aceton vorgelegt. Die Emulsion wurde bei 500 U/min und +35°C im Ölbad 2,5 h gerührt. Es konnte eine Ausbeute von 4,3 g (29 mmol, 92%) Methioninamid via HPLC detektiert werden.

### Beispiel 3: (Vergleich): Hydrolyse von MMP-Aminonitril mit NaOH, Ammoniak und Aceton

Es wurden 17,5 g VE-Wasser, 145 mg NaOH (4 mmol, 0,13 Moleq), 26.5 g einer 32%-igen Ammoniak-Lösung (498 mmol, 16 Moleq), 5,1 g MMP-AN (w/w 80%; 31 mmol, 1,0 Moleq) und 420 mg (7 mmol, 0,23 Moleq) Aceton bei Raumtemperatur vorgelegt. Das Reaktionsgemisch wurde bei 500 U/min und +40°C im Ölbad 1,5 h gerührt. Es konnte eine Ausbeute von 3,9 g (26 mmol, 83%) Methioninamid via HPLC detektiert werden.

### Beispiel 4: Hydrolyse von Methionin-Amid mit TiO₂ zu Methionin und Ammoniak

In einem Rundkolben mit Rückflusskühler wurden Methioninamid (30 mmol, 9.0 g einer 50% wässrige Lösung), Hombikat F01 (1.0 g Pulver) und Wasser (80 mL) eine Stunde bei 85 °C Badtemperatur gekocht. Es konnte eine Ausbeute von 4.43 g (>99%) Methionin via HPLC detektiert werden.

### Beispiel 5: Abtrennen von Dimethylamin und Aceton

Die Reaktionsmischung aus Versuch 1 wurde von oben durch eine mit Raschigringen gefüllte Doppelmantel-Glassäule (Höhe: 40 cm, Innendurchmesser: 3 cm, 105 °C Manteltemperatur) geleitet, während von unten Dampf (4 g/min) eingeleitet wurde. Der Gehalt an Dimethylamin und Aceton nach Durchgang durch die Strippkolonne wurde via GC gemessen. Beide Komponenten waren praktisch vollständig entfernt.

### Beispiele 6 bis 8: Hydrolyse von MMP-Aminonitril mit 4-Piperidon oder primärer Aminbase und Aceton

Methioninnitril (1 Moleq) wurde als 14 gewichtsproz. wässrig-ammoniakalische Lösung (enthaltend 6 Moleq Ammoniak) bei 35 °C vorgelegt und Aceton (1,2 Moleq; außer in Beispiel 6) sowie 1,2 Moleq der jeweils in Tabelle 1 angegebenen Aminbase zugegeben und die Mischung anschließend bei 35 °C 2,5 h lang gerührt.

**Tabelle 1: Hydrolyse von MMP-Aminonitril mit 4-Piperidon oder Aminbase und Aceton**

| **Beispiel** | **Base bzw. Keton** | **Umsatz** | **Selektivität** | **Ausbeute** | **Wieder- Findung** | **MMP-Aus beute(Rück bildung) [%]** |
|---|---|---|---|---|---|---|
| | | **[%]** | **[%]** | **[%]** | **[%]** | |
| 6 | 4-Piperidon ohne Aceton | 67 | 86 | 58 | 89 | <2 |
| 7 (Vergleich) | Spermidin | 53 | 48 | 25 | 92 | 20 |
| 8 (Vergleich) | 1,2-Diaminocyclohexan | 38 | 45 | 17 | 96 | 18 |

Die Umsätze sind mit den primären Aminen Spermidin bzw. 1,2-Diaminocyclohexan als basischer Katalysator in der gegebenen Zeit nicht mehr vollständig, womit auch gleichzeitig eine geringere Selektivität durch eine erhöhte Rate der Nebenreaktionen verbunden ist. Insbesondere zeigt sich bei diesen Vergleichsbeispielen 7 und 8 auch eine hohe Rückbildungsrate von MMP, was aber für ein technisches Verfahren prohibitiv erscheint.
Bei Verwendung von 4-Piperidon als kombiniertem Basen- und Carbonylkatalysator hingegen werden wieder höhere Umsätze und Selektivitäten erreicht, so dass dies bei ausreichender Reaktionszeit und -temperatur entsprechend zu vollständigem Umsatz führen
muss.

### Beispiel 9 (Vergleich): Hydrolyse von MMP-Aminonitril mit NaOH, Ammoniak und 4-Piperidon

Methioninnitril (1 Moleq) wurde als 29 gewichtsproz. wässrig-ammoniakalische Lösung (enthaltend 0,3 Moleq Ammoniak und 17,5 Moleq Wasser) bei einer Starttemperatur von 30 °C vorgelegt und verdünnte NaOH (0,2 Moleq) sowie 4-Piperidon (0,2 Moleq) zugegeben und die Mischung anschließend bei ca. 35 °C 0,25 h lang gerührt. Es wurde eine Ausbeute von 84,6% Methioninamid sowie von 1,9% Methioninnatriumsalz via HPLC detektiert.

### Beispiel 10 (Vergleich): Hydrolyse von MMP-Aminonitril mit NaOH, Ammoniak und Cyclohexanon

Methioninnitril (1 Moleq) wurde als 29 gewichtsproz. wässrig-ammoniakalische Lösung (enthaltend 0,3 Moleq Ammoniak und 17,5 Moleq Wasser) bei einer Starttemperatur von 30 °C vorgelegt und verdünnte NaOH (0,2 Moleq) sowie Cyclohexanon (0,2 Moleq) zugegeben und die Mischung anschließend bei ca .35 °C 0,5 h lang gerührt. Es wurde eine Ausbeute von 78,6% Methioninamid sowie von 2,3% Methioninnatriumsalz via HPLC detektiert.

## Patentansprüche

1. Verfahren zur Herstellung von Methioninamid durch Hydrolyse von Methioninnitril in Gegenwart eines Ketons und eines sekundären Amins als Katalysator.

2. Verfahren zur Herstellung von Methioninamid **gekennzeichnet durch** folgende Schritte:
a. Herstellung von Methioninnitril durch Umsetzung von Methylmercaptopropionaldehyd (MMP) mit Blausäure und Ammoniak und/oder durch Umsetzung des entsprechenden MMP-Cyanhydrins (MMP-CH) mit Ammoniak zu einem Methioninnitril enthaltendem Reaktionsgemisch,
b. optional wenigstens teilweise Abtrennung von Wasser und restlichem Ammoniak aus dem Reaktionsgemisch aus a.,
c. Hydrolyse des Methioninnitrils zum Methioninamid im Reaktionsgemisch aus a. oder b. in Gegenwart eines Ketons und eines sekundären Amins als Katalysator und
d. optional Abtrennung des Ketons und des sekundären Amins aus dem Reaktionsgemisch aus c.

3. Verfahren zur Herstellung von Methionin **gekennzeichnet durch** folgende Schritte:
a. Herstellung von Methioninnitril durch Umsetzung von Methylmercaptopropionaldehyd (MMP) mit Blausäure und Ammoniak und/oder durch Umsetzung des entsprechenden MMP-Cyanhydrins (MMP-CH) mit Ammoniak zu einem Methioninnitril enthaltenden Reaktionsgemisch,
b. optional wenigstens teilweise Abtrennung von restlichem Ammoniak aus dem Reaktionsgemisch aus a.,
c. Hydrolyse von Methioninnitril zum Methioninamid im Reaktionsgemisch aus a. oder b. in Gegenwart eines Ketons und eines sekundären Amins als Katalysator,
d. optional wenigstens teilweise Abtrennung des Ketons und des sekundären Amins aus dem Reaktionsgemisch aus c. und
e. anschließender Hydrolyse des Methioninamids zum Methionin im Reaktionsgemisch aus c. oder d. in Gegenwart eines sauren, basischen oder neutralen Katalysators.

4. Verfahren gemäß Anspruch 1, 2 oder 3, **dadurch gekennzeichnet, dass** bei der Hydrolyse des Methioninnitrils als sekundäres Amin, Dimethylamin, Diethylamin, Dipropylamin, Diisopropylamin, Pyrrolidin oder 4-Piperidon eingesetzt werden.

5. Verfahren gemäß Anspruch 1, 2 oder 3, **dadurch gekennzeichnet, dass** bei der Hydrolyse des Methioninnitrils als Keton Aceton, Methyl-ethylketon, Diethylketon, Methyl-propylketon, Methyl-isopropylketon, Ethyl-propylketon, Cyclopentanon, Cyclohexanon oder 4-Piperidon, vorzugsweise Aceton, Methyl-ethylketon oder 4-Piperidon eingesetzt werden.

6. Verfahren gemäß Anspruch 4 oder 5, **dadurch gekennzeichnet, dass** im Falle der Verwendung von 4-Piperidon als Keton kein weiteres Keton oder im Falle der Verwendung von 4-Piperidon als sekundäres Amin kein weiteres sekundäres Amin verwendet wird.

7. Verfahren gemäß Anspruch 1, 2 oder 3, **dadurch gekennzeichnet, dass** die Hydrolyse des Methioninnitrils bezogen auf das Nitril in Gegenwart von 15 bis 50 Moleq H₂O, 0,1 bis 1,5 Moleq Keton, 0 bis 9 Moleq NH₃ 0,1 bis 2,0 Moleq sekundäres Amin, sowie bei einer Temperatur zwischen 10 und 50°C und bei einer Reaktionszeit zwischen 10 und 180 Minuten durchgeführt wird.

8. Verfahren gemäß Anspruch 2 oder 3, **dadurch gekennzeichnet, dass** bei der Umsetzung gemäß Schritt a. 1 bis 10 Moleq Ammoniak, vorzugsweise 2-8 Moleq Ammoniak und besonders bevorzugt 4-7 Moleq Ammoniak eingesetzt werden bezogen auf die molare Summe aus Methylmercaptopropionaldehyd und MMP-Cyanhydrin.

9. Verfahren gemäß Anspruch 2 oder 3, **dadurch gekennzeichnet, dass** bei der Umsetzung gemäß Schritt a. wäßriger Ammoniak in einer Konzentration von 25 bis 80 Gew.-%, vorzugsweise von 30 bis 60 Gew.-%, eingesetzt wird.

10. Verfahren gemäß Anspruch 2 oder 3, **dadurch gekennzeichnet, dass** die Abtrennung von restlichem Wasser und Ammoniak gemäß Schritt b. bei einem Druck von 0,01 bis 3 bara und einer Temperatur von 20 bis 70 °C, vorzugsweise von 0,1 bis 1 bara und einer Temperatur von 25 bis 60 °C erfolgt.

11. Verfahren gemäß Anspruch 2 oder 3, **dadurch gekennzeichnet, dass** die Abtrennung des Ketons und des sekundären Amins gemäß Schritt d. bei einem Druck von 0,001 bis 1 bara und einer Temperatur von 20 bis 100 °C, vorzugsweise von 0,01 bis 0,9 bar und einer Temperatur von 25 bis 80 °C erfolgt.

12. Verfahren nach den Ansprüchen 2, 3 oder 11, **dadurch gekennzeichnet, dass** das gemäß Schritt d. abgetrennte Keton und sekundäre Amin zur Hydrolyse des Methioninnitrils in Schritt c. zurückgeführt werden.

13. Verfahren gemäß Anspruch 3, **dadurch gekennzeichnet, dass** bei der Hydrolyse des Methioninamids gemäß Schritt e. als saurer Katalysator eine starke Mineralsäure, vorzugsweise Salzsäure, Schwefelsäure oder Phosphorsäure verwendet wird.

14. Verfahren gemäß Anspruch 3, **dadurch gekennzeichnet, dass** bei der Hydrolyse des Methioninamids gemäß Schritt e. als basischer Katalysator ein Alkali- oder Erdalkali- Base, vorzugsweise NaOH, KOH, Mg(OH)₂, Ca(OH)₂ oder Ba(OH)₂, verwendet wird.

15. Verfahren gemäß Anspruch 3, **dadurch gekennzeichnet, dass** bei der Hydrolyse des Methioninamids gemäß Schritt e. als neutraler Katalysator Titandioxid, verwendet wird.

16. Verfahren nach einem oder mehreren der Ansprüche 3 bis 15, **dadurch gekennzeichnet, dass** die Hydrolyse des Methioninamids gemäß Schritt e. bei Temperaturen von 70-180°C, vorzugsweise von 100-140°C durchgeführt wird.

17. Verfahren zur Herstellung eines α-Aminosäureamids durch Hydrolyse des entsprechenden α-Aminosäurenitrils in Gegenwart eines Ketons und eines sekundären Amins als Katalysator.

18. Verfahren gemäß Anspruch 17, **dadurch gekennzeichnet, dass** es sich bei dem α-Aminosäureamid um das jeweilige Amid der α-Aminosäure Alanin, Serin, Homoserin, Threonin, Valin, Leucin, Isoleucin, Lysin, Methionin Tryptophan, Histidin, Phenylalanin, Glycin oder Tyrosin handelt.

19. Verfahren gemäß Anspruch 17 oder 18, **dadurch gekennzeichnet, dass** bei der Hydrolyse des α-Aminosäurenitrils als sekundäres Amin, Dimethylamin, Diethylamin, Dipropylamin, Diisopropylamin, Pyrrolidin oder 4-Piperidon eingesetzt werden.

20. Verfahren gemäß einem der Ansprüche 17 bis 19, **dadurch gekennzeichnet, dass** bei der Hydrolyse des α-Aminosäurenitrils als Keton Aceton, Methyl-ethylketon, Diethylketon, Methyl-propylketon, Methyl-isopropylketon, Ethyl-propylketon, Cyclopentanon, Cyclohexanon oder 4-Piperidon, vorzugsweise Aceton, Methyl-ethylketon oder 4-Piperidon eingesetzt werden.

21. Verfahren zur Herstellung einer α-Aminosäure umfassend
einen ersten Schritt a) der Hydrolyse des entsprechenden α-Aminosäurenitrils zum jeweiligen α-Aminosäureamid in Gegenwart eines Ketons und eines sekundären Amins als Katalysator und
einen zweiten Schritt b) der Weiterhydrolyse des α-Aminosäureamids zur entsprechenden α-Aminosäure.
